# EUROPÄISCHE PATENTSCHRIFT

(11) **EP 2 458 367 B1**
(45) Veröffentlichungstag und Bekanntmachung des Hinweises auf die Patenterteilung: **05.08.2015**
(21) Anmeldenummer: 10192601.2
(22) Anmeldetag: 25.11.2010
(51) Int. Cl.: G01N 13/00, G01N 15/02, G01N 15/14, G01N 33/15, G01N 21/90

(54) **Vorrichtung und Verfahren zur Erkennung fester Substanzen in einer flüssigen Phase**
Device and method for recognising solid substances in a liquid phase
Dispositif et procédé de reconnaissance de substances solides dans une phase liquide

(43) Veröffentlichungstag der Anmeldung: 30.05.2012
(73) Patentinhaber: Mettler-Toledo AG, 8606 Greifensee (CH)
(72) Erfinder: Engelhardt, Matthias, 8640, Rapperswil (CH); Schuster, Guido, 8712, Stäfa (CH); Meyer, Michalis, 8905, Islisberg (CH); Nufer, Bruno, 8308, Illnau (CH)

(56) Entgegenhaltungen:
- WO-A1-03/098199
- WO-A1-2006/108908
- WO-A2-2006/084283
- US-A- 6 006 590
- US-A1- 2004 138 827
- US-A1- 2004 168 529
- US-A1- 2005 003 550
- US-A1- 2005 225 766
- US-A1- 2009 207 691
- US-B1- 6 170 980
- US-B2- 6 866 823

## Beschreibung

Die vorliegende Erfindung bezieht sich auf eine Vorrichtung und ein Verfahren zur Erkennung fester Substanzen in einer flüssigen Phase, wobei die feste Substanz und die flüssige Phase als Gemisch in einem zumindest teilweise transparenten Behälter eingeschlossen sind.

In vielen Bereichen der Industrie, insbesondere in der Produktion, Forschung und Entwicklung sind periodisch Flüssigkeiten auf das Vorhandensein fester Substanzen, beispielsweise Partikel von Pulvern, Schwebestoffe und dergleichen zu untersuchen.

Ferner müssen feste Substanzen in pastenförmiger, pulverförmiger oder granulatförmiger Form in einer flüssigen Phase aufgelöst werden. Eine oder mehrere feste Substanzen werden in einen Behälter eindosiert und die flüssige Phase, meistens Wasser oder ein organisches Lösungsmittel wie Ethanol, entweder nach Gewicht oder Volumen beigegeben. Die Konzentration der durch den Auflösungsprozess entstandenen Lösung hängt entscheidend davon ab, ob alle festen Substanzteile aufgelöst wurden oder nicht.

Zur Untersuchung von Flüssigkeiten auf feste Substanzen und zur Kontrolle von Auflösungsprozessen werden verschiedene Methoden und Systeme eingesetzt. Die einfachste Methode beschränkt sich darauf, die zu untersuchende Flüssigkeit einer visuellen Kontrolle zu unterziehen. Bei Auflösungsprozessen kann das im Behälter befindliche Gemisch aus fester Substanz und flüssiger Phase solange gerührt werden, bis die periodisch durchgeführte visuelle Kontrolle ergibt, dass keine festen Substanzteile mehr sichtbar sind. Eine weitere Methode basiert auf Erfahrungswerten, wie lange das Gemisch aus einer bestimmten festen Substanz und einem bestimmten Lösungsmittel gerührt werden muss, bis die Substanz vollständig aufgelöst ist.

Diese Methoden sind ineffizient, da die visuelle Kontrolle sehr arbeitsintensiv ist und das Festlegen der Rührzeit anhand von Erfahrungswerten gegebenenfalls dazu führt, dass der Auflösungsprozess übermässig lange dauert. Zudem ist bei der visuellen Kontrolle die Fehleranfälligkeit hoch, da in den meisten Prozessschritten Bedienungspersonen direkt involviert sind.

Um den Einfluss der Bedienungspersonen zu minimieren, wird in der US 5,152,180 A eine Vorrichtung zur Überwachung von Auflösungsprozessen fester Substanzen in einer flüssigen Phase vorgeschlagen, wobei die feste Substanz und die flüssige Phase als Gemisch in einen Behälter mit einem Rührwerk eingefüllt sind. Um den Auflösungsprozess zu überwachen, werden Signale im breitbandigen Ultraschall-Frequenzbereich von einem Sender in die flüssige Phase ausgesendet und mittels eines Detektors die Resonanzfrequenz der flüssigen Phase gemessen. Mit zunehmender Auflösung der festen Substanz ändert sich auch die Resonanzfrequenz, wodurch der Abschluss des Auflösungsprozesses detektierbar ist. Wenn alle feste Substanz aufgelöst ist, ändert sich die Resonanzfrequenz nicht mehr und die Vorrichtung nutzt diesen stabilen Zustand der Resonanzfrequenz, das Ende des Auflösungsprozesses anzuzeigen.

Die vorangehend beschriebene Vorrichtung hat den Nachteil, dass die Messung der Resonanzfrequenz durch das Rührwerk negativ beeinflusst werden kann. Ferner können Substanzpartikel oberhalb des Flüssigkeitsspiegels an der Wand des Behälters anhaften und nicht in Lösung gehen. Des Weiteren sind der Sender und der Empfänger in der Gehäusewand des Behälters eingelassen und gelangen somit in Berührung mit der Lösung.

WO 03/098199 A1 behandelt ein Nachweissystem zur Messung der Freisetzung eines Wirkstoffes aus einer pharmazeutischen Darreichungsform.

US 2004/0138827 A1 behandelt ein System und Verfahren zur Erleichterung von Kristallisationsstudien.

US 2004/0168529 A1 behandelt Verfahren und Systeme zur Bestimmung von Löslichkeitsprofilen von Probenmaterialien.

Die Aufgabe der vorliegenden Erfindung ist deshalb, eine Vorrichtung und ein Verfahren zu schaffen, durch welche die Erkennung fester Substanzen in einer flüssigen Phase ermöglicht wird oder der Auflösungsprozess eines Gemischs aus mindestens einer festen Substanz und einer flüssigen Phase überwacht werden kann, ohne dabei die Vorrichtung zu kontaminieren.

Diese Aufgabe wird durch die erfindungsgemässe Vorrichtung und das erfindungsgemässe Verfahren zur Erkennung fester Substanzen in einer flüssigen Phase gelöst.

Eine Vorrichtung zur Erkennung fester Substanzen in einer flüssigen Phase umfasst eine Kamera zur Erzeugung digitalisierter Aufnahmen und eine Prozessoreinheit. Diese digitalisierten Aufnahmen werden als Array von Pixel mit ihren zugehörenden Pixelwerten erfasst. Die feste Substanz und die flüssige Phase sind als Gemisch in einem zumindest teilweise transparenten Behälter eingeschlossen. Ein teilweise transparenter Behälter bedeutet im Zusammenhang mit der vorliegenden Erfindung, dass mindestens eine Stelle der Wände die den Behälter bilden für elektromagnetische Wellen absorptionsarm durchdringbar ist. Die übrigen Stellen der Wände des Behälters können beliebig ausgestaltet oder den erfindungsgemässen Zweck unterstützend ausgebildet sein. Die für die Vorrichtung verwendeten Teile müssen somit auf die zu verwendenden elektromagnetischen Wellen abgestimmt sein. Sofern beispielsweise Tageslicht verwendet wird und dies zur Ausleuchtung des Gemischs ausreicht, muss nicht zwingend eine Beleuchtungsvorrichtung vorhanden sein. Wie weiter unten ausgeführt, ist jedoch eine Beleuchtungsvorrichtung zur Erreichung optimaler Ergebnisse empfehlenswert.

Die Vorrichtung weist ferner eine Bewegungsvorrichtung zur Aufnahme des Behälters auf, mittels welcher Bewegungsvorrichtung der Behälter bewegt werden kann um dem Gemisch dadurch kinetische Energie zuzuführen. Die Zuführung kinetischer Energie hat die Hauptaufgabe, das im Behälter eingeschlossene Gemisch in Bewegung zu halten. Üblicherweise bleibt das Gemisch beziehungsweise die Lösung, auch nach dem Abschalten der Bewegungsvorrichtung noch eine gewisse Zeit in Bewegung. Diese Bewegungen des Gemisches werden wie weiter unten beschrieben, zur Erkennung fester Substanz in einer flüssigen Phase ausgenutzt.

Ferner kann die Zuführung kinetischer Energie noch zwei Nebenaufgaben erfüllen. Erstens wird die feste Substanz infolge der Durchmischung und der vorhandenen turbulenten Strömungen in der flüssigen Phase rascher aufgelöst. Weil der Behälter geschlossen ist, kann dieser mittels der Bewegungsvorrichtung beliebig gedreht und geschüttelt werden, so dass zweitens alle festen Substanzpartikel mit der flüssigen Phase in Berührung kommen und gegebenenfalls aufgelöst werden.

Weiterhin ist der Behälter im Betriebszustand der Vorrichtung mit einer Aufnahme der Bewegungsvorrichtunglösbar verbunden. Die Aufnahme der Bewegungsvorrichtung ist über eine Aufnahmewelle mit einem Antrieb verbunden. Mittels des Antriebs an der Aufnahmewelle sind oszillierende Bewegungen in deren Längsrichtung erzeugbar.

Als Prozessoreinheit wird im Rahmen der vorliegenden Schrift jede Vorrichtung verstanden, die digitale Daten verarbeiten und speichern kann, beispielsweise ein Prozessleitsystem, ein an die übrigen Teile der Vorrichtung anschliessbarer Computer oder Laptop, aber auch integrierte Lösungen wie eine in der Bewegungsvorrichtung angeordnete Platine mit einem Prozessor, eine in die Kamera integrierte Recheneinheit oder einem FPGA (Field Programmable Gate Array), mit Speichermodulen und einer damit verbundenen Ein-/Ausgabeeinheit.

Die Kamera ist zur bildlichen Erfassung des bewegten Gemischs auf eine transparente Stelle des Behälters gerichtet. Mittels der Kamera werden während einer Aufnahmephase digitalisierte Aufnahmen des bewegten Gemischs erzeugt und an die Prozessoreinheit übermittelt. Der Zweck der Erzeugung digitalisierter Aufnahmen besteht darin, im nachfolgenden Auswertungsprozess den statischen Bildanteil vom dynamischen Bildanteil zu trennen. Der statische Bildanteil umfasst alle Teile des Behälters und der Umgebung des Behälters, der dynamische Bildanteil umfasst das sich im Behälter bewegende Gemisch.

Am einfachsten ist die Erzeugung für den Auswertungsprozess geeigneter digitalisierter Aufnahmen realisierbar, wenn die Kamera ortsfest installiert ist und der Behälter periodisch in eine Ruhephase gebracht wird. Selbstverständlich kann die Kamera auch mit dem Behälter fest verbunden sein oder diesem in der Aufnahmephase synchron nachgeführt werden, so dass sie während der Aufnahmephase jeder Bewegung des Behälters nachfolgt. Durch diese zweite Ausführung kann die Aufnahmephase nicht nur in den Ruhephasen des Behälters, sondern auch während der Zuführung kinetischer Energie erfolgen. Als dritte Ausführung kann der Auslösezeitpunkt der Kamera mit einer bestimmten Position des Behälters gekoppelt sein, so dass der Behälter immer in scheinbar derselben Lage zu seiner Umgebung aufgenommen wird. Eine vierte Möglichkeit besteht darin, beliebige Aufnahmen des sich bewegenden Behälters zu machen, wobei der nachfolgende Auswertungsprozess erheblich höhere Rechenleistung erfordert und deshalb nicht empfehlenswert ist.

In der Aufnahmephase wird von der Kamera mindestens eine Serie, umfassend mindestens zwei digitalisierte Aufnahmen des bewegten Gemisches erzeugt. Damit diese digitalisierten Aufnahmen während des weiteren Auswertungsprozesses vorhanden sind, werden diese vorzugsweise in einem Zwischenspeicher abgespeichert. Aus mindestens zwei der digitalisierten Aufnahmen kann ein Analysebild des bewegten Gemischs erzeugt werden.

In einer ersten Ausführung der Erfindung wird das Analysebild mittels der Prozessoreinheit aus zwei digitalisierten Aufnahmen derselben Serie durch die Subtraktion der Pixelwerte einer ersten Aufnahme von den Pixelwerten einer zweiten Aufnahme erzeugt. Auf dem Analysebild sind die sich bewegenden festen Substanzteile des Gemischs als Kontrastflächen beziehungsweise als unterschiedliche Pixelwerte erkennbar.

In einer zweiten Ausführung der Erfindung wird ein Durchschnittsbild aus einer Serie vorangehender digitalisierter Aufnahmen berechnet. Anschliessend wird das Analysebild durch die Subtraktion der Pixelwerte des Durchschnittsbildes von den Pixelwerten einer digitalisierten Aufnahme erzeugt. Auch auf diesem Analysebild sind die sich bewegenden festen Substanzteile des Gemischs als Kontrastflächen beziehungsweise als unterschiedliche Pixelwerte erkennbar.

In einer dritten Ausführung der Erfindung wird das Analysebild mittels einer Bewegungsschätzung der einzelnen Pixel mindesten zweier digitalisierter Aufnahmen erzeugt, wobei durch die Bewegungsschätzung alle Pixel als statischer Bildanteil definiert sind, deren Bewegungsvektoren in den Aufnahmen zueinander 0 sind. Das heisst, dass mit erheblich grösserem Rechenaufwand die Positionen der einzelnen Pixel und deren Pixelwerte mit den umliegenden Pixel und deren Pixelwerten verglichen werden muss. Auch auf diesem Analysebild sind die als dynamischer Bildanteil sich bewegenden festen Substanzteile des Gemischs als Kontrastflächen beziehungsweise als unterschiedliche Pixelwerte erkennbar.

Die Kamera kann zum Projizieren des Bildes des bewegten Gemisches auf deren Bildebene eine Linse oder eine Lochblende umfassen. Die Bildebene ist mit einer zweidimensionalen Anordnung von Strahlungssensoren versehen. Der besseren Übersicht wegen wird diese zweidimensionale Anordnung von Strahlungssensoren nachfolgend als Sensorelement bezeichnet. Es gibt eine Vielzahl solcher Sensorelemente für elektromagnetische Wellen, beispielsweise CCD-Sensoren, Active-Pixel-Sensoren, Focal-Plane-Arrays, Sensorelemente für Röntgenstrahlung und dergleichen mehr. Das auf die Bildebene projizierte, zweidimensionale Bild wird von der Kamera in der Form eines zweidimensionalen Arrays von Bildelementen beziehungsweise Pixel erfasst und die für jedes Pixel erzeugten digitalen Daten stellen die Farbe und Luminanz dar. Im Allgemeinen werden die Beiträge des Lichts, das von den verschiedenen Punkten des bewegten Gemischs reflektiert wird, zu dem Pixelwert, der die Farbe und die Intensität eines bestimmten Pixels darstellt. Bei der Verwendung einer Graustufen-Kamera entfällt logischerweise die Farbangabe.

Da die Kamera vorzugsweise den gesamten Behälter bildlich erfasst, können auch Bereiche des Behälters abgebildet sein, die eine sinnvolle Auswertung erschweren. Beispielsweise kann der Behälter eine Flasche mit zylindrischem Querschnitt sein, wobei durch die Krümmung des Glases der Randbereich des Behälters kaum eine auswertbare Abbildung des bewegten Gemischs zulässt. Grundsätzlich ist aber jeder verschliessbare Behälter mit beliebiger Form verwendbar, vorausgesetzt, er weist mindestens eine transparente Stelle auf.

Die Vorrichtung und der Behälter können zur reinen Analyse genutzt werden, das heisst, dass eine unbekannte Flüssigkeit auf das Vorhandensein von fester Substanz untersucht werden soll oder ein möglicher Auflösungsprozess bereits vorher stattgefunden hat und die zu untersuchende, fertige Lösung in den Behälter eingefüllt und dieser in die erfindungsgemässe Vorrichtung eingesetzt wird. Sinnvollerweise wird aber die Lösung im Behälter hergestellt. Die Ausgestaltung des Behälters kann dem entsprechend auf den Verwendungszweck als Misch- und Analysebehälter optimiert sein, so dass die Innenkontur des Behälters den Auflösungsprozess der festen Substanz in der flüssigen Phase infolge der eingebrachten kinetischen Energie optimal unterstützt. Dies ist beispielsweise durch Verengungen in Teilabschnitten des Behälters möglich, aber auch durch die Vermeidung von Ecken, in denen sich "Totwasserbereiche" bilden und den Auflösungsprozess der dort angesammelten festen Substanz hemmen können. Die Wände des Behälters können zudem auf die optische Analysemethode optimiert sein. Beispielsweise können in der Wand ausgebildete verzerrungsfreie Bereiche ausgebildet sein, auf welche die Kamera zur Erfassung der digitalisierten Aufnahmen gerichtet ist. Auch fokussierende oder streuende Linsen können in der Wand ausgebildet sein. Grundsätzlich ist für die Ausbildung des transparenten Bereichs jedes transparente Material geeignet, insbesondere Glas und transparente Kunststoffe. Die Wahl des geeigneten Materials hängt im Wesentlichen von der Wahl der flüssigen Phase beziehungsweise dem Lösungsmittel und den Eigenschaften der festen Substanz in ihrer festen und/oder gelösten Form ab. Ferner kann der Behälter eine weitere transparente Stelle oder eine Stelle mit kleiner Opazität aufweisen, um die Ausleuchtung des zu erfassenden Gemischs optimal zu unterstützen.

Um zu vermeiden, dass zur Auswertung unbrauchbare Bereiche mit ausgewertet werden, wird in einer Weiterbildung des Erfindungsgegenstandes aus dem Analysebild mindestens ein Auswertungsbereich ausgewählt. Anschliessend wird der Auswertungsbereich auf Kontrastflächen hin untersucht. Selbstverständlich kann auch je ein Ausschnitt aus der ersten Aufnahme und der zweiten Aufnahme ausgewählt werden und aus diesen Ausschnitten ein den Auswertungsbereich ergebendes Analysebild erzeugt werden.

Der mindestens eine Auswertungsbereich kann ferner in Segmentflächen gleicher oder unterschiedlicher Grösse segmentiert sein. Dies hat den Vorteil, dass die einzelnen Segmentflächen separat ausgewertet werden können und dadurch sequentiell kleinere Datenmengen verarbeitet werden müssen. Ferner können durch die Segmentierung in der nachfolgenden statistischen Bewertung lokale Extreme gesucht werden, wodurch die Vorrichtung betreffend die Detektierung von Partikeln empfindlicher wird.

Die Untersuchung erfolgt dadurch, dass innerhalb eines Analysebildes oder einer Segmentfläche die Häufigkeitsverteilung der Pixelwerte ausgewertet wird. Zur Eliminierung kamerabedingten Rauschens wird eine vorbestimmte Bandbreite von Pixelwerten der Häufigkeitsverteilung in einer weiteren Auswertung nicht berücksichtigt. Die vorbestimmte Bandbreite von Pixelwerten kann beispielsweise mit einer klaren Referenz-Lösung ohne ungelöste Substanz bestimmt werden.

Die Vorrichtung kann ferner eine Quelle aufweisen die elektromagnetische Wellen aussendet, die auf das Sensorelement der Kamera abgestimmt ist. Da die meisten handelsüblichen Kameras im Bereich des sichtbaren Lichts arbeiten und deshalb niedrige Beschaffungskosten verursachen, weist die Vorrichtung vorzugsweise mindestens eine flächige Beleuchtungsvorrichtung auf. Der Behälter, durch die Bewegungsvorrichtung bewegt, kann beispielsweise zwischen der flächigen Beleuchtungsvorrichtung und der Kamera angeordnet sein. Dadurch wird das Gemisch von der flächigen Beleuchtungsvorrichtung hinterleuchtet. Dies bedingt natürlich, dass die der transparenten Stelle gegenüberliegende Wand des Behälters ebenfalls transparent oder zumindest eine gewisse Lichtdurchlässigkeit aufweisend, ausgebildet ist. Die flächige Lichtquelle kann selbstverständlich auch in einer beliebigen räumlichen Lage auf den Behälter gerichtet sein, so dass nur durch die Partikel der festen Substanz gestreutes und/oder abgelenktes Licht auf die Kamera trifft. Zusätzlich oder anstelle der flächigen Beleuchtungsvorrichtung kann die Vorrichtung mindestens eine punktförmige Beleuchtungsvorrichtung, einen sogenannten Spot aufweisen. Die elektromagnetischen Wellen beziehungsweise Lichtwellen der mindestens einen punktförmigen Beleuchtungsvorrichtung sind auf die von der Kamera zu erfassende, transparente Stelle des Behälters gerichtet. Die von den Partikeln des Gemischs reflektierten, abgelenkten oder teilabsorbierten Lichtwellen werden vom Sensorelement der Kamera als helle und dunkle Kontrastflächen erfasst und ergeben bezogen auf die Pixelwerte der flüssigen Phase von diesen unterscheidbare Pixelwerte.

Wie weiter oben ausgeführt, wird der Behälter durch die Bewegungsvorrichtung bewegt. Die Bewegungen können in beliebiger Richtung erfolgen, beispielsweise kann die Bewegungsvorrichtung den Behälter in seiner Längsrichtung oszillierend bewegen, schwenken, rotieren oder den Behälter mehrmals über Kopf drehen. Wichtig ist nur, dass möglichst alle Substanzpartikel in Bewegung versetzt werden können, damit sie sich relativ zum Behälter bewegen und damit erkennbar sind. Eine sichere Kontrolle, ob in der flüssigen Phase feste Substanz vorhanden ist, ist dann gegeben, wenn kein Partikel an der Innenseite der Behälterwand anhaftet. Damit alle festen Substanzpartikel durch die flüssige Phase sicher erreicht werden können und nicht beispielsweise in einem unbenetzten Bereich der Behälterwand anhaften, kann die Bewegungsvorrichtung derart ausgestaltet sein, dass der Behälter auch um seine Mittellängsachse drehbar ist. Die Bewegungsvorrichtung kann auch für einen Auflösungsprozess eingesetzt werden. Um die Bewegungsvorrichtung zu schonen, kann der Auflösungsprozess oder zumindest der grösste Teil des Auflösungsprozesses auch schon vor dem Einsetzen des Behälters in die erfindungsgemässe Vorrichtung erfolgen.

Zur Erkennung fester Substanzen in einer flüssigen Phase kann das nachfolgende Verfahren eingesetzt werden. Dazu müssen erfindungsgemäss die feste Substanz und die flüssige Phase als Gemisch in einem zumindest teilweise transparenten Behälter eingeschlossen sein. Das erfindungsgemässe Verfahren weist zumindest die Schritte auf, dass
- der Behälter bewegt und dem Gemisch dadurch kinetische Energie zugeführt wird,
- mittels der Kamera in einer Aufnahmephase mindestens eine Serie mit mindestens zwei digitalisierten Aufnahmen des im Behälter bewegten Gemischs als Arrays von Pixel mit ihren zugehörenden Pixelwerten gemacht werden,
- aus den Arrays von Pixel mit ihren zugehörenden Pixelwerten ein Analysebild berechnet wird, wobei die sich bewegenden festen Substanzteile als Kontrastflächen beziehungsweise unterschiedliche Pixelwerte erkennbar sind, und
- das Analysebild auf das Vorhandensein von Kontrastflächen beziehungsweise unterschiedliche Pixelwerte untersucht wird.

Dabei ist der Behälter im Betriebszustand der Vorrichtung mit einer Aufnahme der Bewegungsvorrichtung lösbar verbunden. Die Aufnahme der Bewegungsvorrichtung ist über eine Aufnahmewelle mit einem Antrieb verbunden. Mittels des Antriebs an der Aufnahmewelle sind oszillierende Bewegungen in deren Längsrichtung erzeugbar.

Das Verfahren kann durch den weiteren Schritt ergänzt werden, dass aus dem Analysebild mindestens ein Auswertungsbereich ausgewählt wird. Selbstverständlich kann auch je ein Ausschnitt aus zwei digitalisierten Aufnahmen derselben Serie ausgewählt werden und aus diesen Ausschnitten ein den Auswertungsbereich ergebendes Analysebild erzeugt werden.

Ferner ist das erfindungsgemässe Verfahren durch die weiteren Schritte ergänzbar, dass das Analysebild oder der mindestens eine Auswertungsbereich in Segmentflächen gleicher oder verschiedener Grösse segmentiert wird und jede Segmentfläche auf das Vorhandensein von Kontrastflächen beziehungsweise auf unterschiedliche Pixelwerte untersucht wird.

Die Untersuchung des Analysebildes oder der Segmentfläche kann dadurch erfolgen, dass deren Häufigkeitsverteilung der Pixelwerte ausgewertet wird und zur Eliminierung kamerabedingten Rauschens eine vorbestimmte Pixelwert- Bandbreite der Häufigkeitsverteilung in einer weiteren Auswertung nicht berücksichtigt wird.

Das Verfahren kann die weiteren Schritte aufweisen, dass die Anzahl Segmentflächen festgestellt wird, deren Häufigkeitsverteilung von der vorbestimmten Pixelwert-Bandbreite abweichende Pixelwerte aufweisen, wobei diese Anzahl der Segmentflächen ein Mass für die vorhandene Menge fester Substanz und/oder für den Fortschritt des Auflösungsprozesses darstellt. Alternativ dazu kann auch die Anzahl der Pixelwerte ermittelt werden, die ausserhalb der Bandbreite eine vordefinierte Schwelle überschreiten. Je weniger Segmentflächen abweichende Pixelwerte aufweisen, desto weniger Partikel sind in der Lösung enthalten. Wenn nur ein Ausschnitt der Lösung betrachtet wird, sind vorzugsweise mehrere Serien von Aufnahmen zu machen und diese wie vorangehend beschrieben zu verarbeiten und auszuwerten, um eine zuverlässige Aussage über den Zustand des zu untersuchenden Gemischs machen zu können. Zwischen den Serien kann dem Gemisch mittels der Bewegungsvorrichtung erneut kinetische Energie zugeführt werden.

Eine genauere Aussage ist durch die weiteren Schritte erzielbar, dass die Segmentflächen, deren Häufigkeitsverteilung von der vorbestimmten Pixelwert-Bandbreite abweichende Pixelwerte aufweisen, nach einem vorgegebenen Modus gewichtet und die gewichteten Werte der Segmentflächen addiert werden, wobei die Summe der Werte ebenfalls ein Mass für die vorhandene Menge fester Substanz und/oder für den Fortschritt des Auflösungsprozesses darstellt. Durch die Gewichtung werden beispielsweise sehr dunkle und sehr helle Kontrastflächen stärker berücksichtigt, weil diese auf grosse Partikel hinweisen. Durch die Grösse der Partikel werden der Auflösungsprozess und insbesondere die dazu benötigte Zeit bekanntlich stark beeinflusst.

Die erfindungsgemässe Vorrichtung und das erfindungsgemässe Verfahren werden im Folgenden anhand von Beispielen und mit Bezugnahme auf die Zeichnungen näher erläutert. Darin zeigen:
- Figur 1: in schematischer Darstellung eine Vorrichtung zur Erkennung einer festen Substanz in einer flüssigen Phase, wobei die Vorrichtung eine Bewegungsvorrichtung für einen Behälter, eine Kamera, eine Beleuchtungsvorrichtung und eine Prozessoreinheit aufweist;
- Figur 2: eine schematische Darstellung der wesentlichen Schritte der Bildung eines Analysebildes und dessen Segmentierung;
- Figur 3: eine erste Häufigkeitsverteilung von Pixelwerten einer Segmentfläche ohne Kontrastflächen in der Darstellung eines Histogramms;
- Figur 4: eine zweite Häufigkeitsverteilung von Pixelwerten einer Segmentfläche mit Kontrastflächen in der Darstellung eines Histogramms;
- Figur 5: ein Flussdiagramm der wesentlichen Verfahrensschritte des erfindungsgemässe Verfahrens;
- Figur 6: ein Flussdiagramm des erfindungsgemässen Verfahrens mit weiteren, die Auswertung betreffenden Verfahrensschritten.

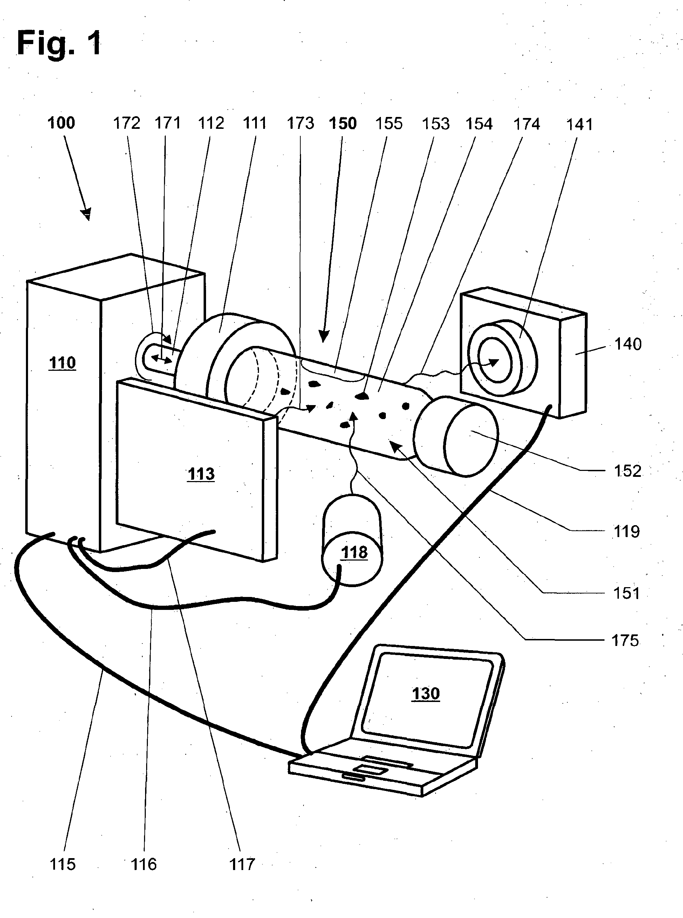

Die Figur 1 zeigt in schematischer Darstellung eine erfindungsgemässe Vorrichtung 100 zur Erkennung einer festen Substanz 153 in einer flüssigen Phase 154. Die Vorrichtung 100 weist eine Bewegungsvorrichtung 110 für einen Behälter 150 auf und beinhaltet ferner eine Kamera 140, mindestens eine Beleuchtungsvorrichtung 113, 118 und eine Prozessoreinheit 130. Die Beleuchtungsvorrichtung 113, 118 ist nicht zwingend erforderlich, wenn die feste Substanz 153 auch ohne diese durch die Kamera 140 erfasst werden kann. Die feste Substanz 153 und die flüssige Phase 154 sind als Gemisch in einem Behälter 150 verfüllt. Wie angedeutet, ist der Behälter 150 nicht randvoll gefüllt, es ist noch ein kleines Gasvolumen 155 mit eingeschlossen. Der Behälter 150 weist einen Behälterverschluss 152 und einen flaschenförmigen Körper auf, welcher aus transparentem Material, beispielsweise Glas oder Kunststoff gefertigt ist. Demzufolge ist der flaschenförmige Körper auch die transparente Stelle 151 des Behälters 150. Der Behälter 150 ist im Betriebszustand der Vorrichtung 100 in seiner Längsrichtung horizontal angeordnet und mit einer Aufnahme 111 der Bewegungsvorrichtung 110 lösbar verbunden. Die Aufnahme 111 kann beispielsweise ein Dreibackenfutter oder eine Spannhülse sein, in welche ein Teilbereich des Behälters 150 eingespannt werden kann. Selbstverständlich sind auch anders ausgestaltete Aufnahmen 111 einsetzbar, wobei deren Ausführung und Funktionsweise im Belieben des Fachmanns liegen und auf den oder die zu verwendenden Behälter 150 abgestimmt sind. Die Aufnahme 111 ist über eine Aufnahmewelle 112 mit einem nicht dargestellten Antrieb verbunden, wobei der Antrieb an der Aufnahmewelle 112 oszillierende Bewegungen 171 in deren Längsrichtung erzeugen kann. Ferner kann durch den Antrieb die Antriebswelle 112 auch in eine Drehbewegung 172 um ihre Mittellängsachse versetzt werden. Dadurch kann die flüssige Phase 154 jeden Bereich des Innenraums des Behälters 150 erreichen, auch Partikel der festen Substanz 153, welche im Bereich des Gasvolumens 155 an der Wand des Behälters 150 anhaften.

Diese Bewegungen 171, 172, welche auch unabhängig voneinander erzeugt werden können, werden von der Aufnahmewelle 112 auf die Aufnahme 111 und von dieser auf den mit der Aufnahme 111 verbundenen Behälter 150 übertragen. Da die flüssige Phase 154 und die feste Substanz 153 vollständig vom Behälter 150 umschlossen sind, wird die kinetische Energie der Bewegungen 171, 172 auf die flüssige Phase 154 übertragen, so dass die feste Substanz 153 in der flüssigen Phase 154 herumwirbelt. Dieser Effekt wird durch das Vorhandensein des Gasvolumens 155 noch verstärkt, da sich die flüssige Phase 154 durch den hohen Dichteunterschied zwischen Gas und Flüssigkeit wesentlich besser bewegen lässt als wenn kein Gasvolumen 155 vorhanden wäre. Durch dieses Herumwirbeln kann sich die feste Substanz 153 viel schneller in der flüssigen Phase 154 auflösen.

Wenn der Antrieb der Bewegungsvorrichtung 110 ausgeschaltet wird und der Behälter 150 sowie die Kamera 140 somit in einer Aufnahmephase sind, weist das Gemisch über die nachfolgende Zeit betrachtet, in abklingender Weise kinetische Energie auf, welche durch stetig langsamer werdende, herumwirbelnde feste Substanz 153 optisch erfassbar ist. Der Effekt der optisch wahrnehmbaren Bewegungen kann zur Erkennung fester Substanz in der flüssigen Phase beziehungsweise zur Überwachung eines Auflösungsprozesses eingesetzt werden, wenn in der Aufnahmephase durch die Kamera 140 mindestens eine Serie von mindestens zwei Aufnahmen des bewegten Gemischs aufgenommen werden.

Die Qualität der Aufnahmen hängt im Wesentlichen von der richtigen Beleuchtung der transparenten Stelle 151 ab. Der Behälter 150 ist im vorliegenden Beispiel deshalb zwischen einer flächigen Beleuchtungsvorrichtung 113 und der Kamera 140 angeordnet, so dass die Lichtwellen 173, 174 der flächigen Beleuchtungsvorrichtung 113 die transparente Stelle 151 des Behälters 150 und des darin eingeschlossenen Gemischs durchdringen und vom Objektiv 141 der Kamera 140 erfasst werden können. Ein Teil der Lichtwellen 173, 174 wird von der festen Substanz 153 absorbiert, reflektiert oder abgelenkt, wodurch auf den Aufnahmen Kontrastflächen entstehen die heller oder dunkler erscheinen, als die Grundfläche der Aufnahme, welche durch die die flüssige Phase 153 durchdringenden Lichtwellen 173, 174 erzeugt wird.

Um die transparente Stelle 151 beziehungsweise das bewegte Gemisch noch besser auszuleuchten, kann zusätzlich zur flächigen Beleuchtungsvorrichtung 113 oder anstelle dieser, auch eine punktförmige Beleuchtungsvorrichtung 118 beziehungsweise ein Spot verwendet werden. Dessen Lichtwellen 175 sind vorzugsweise winklig zur Bildebene der Kamera 140 ausgerichtet, um die Seitenflächen der festen Substanz 153 besser auszuleuchten.

Zur Steuerung der Vorrichtung und des Verfahrens zur Überwachung von Auflösungsprozessen fester Substanzen 153 in einer flüssigen Phase 154 ist eine Prozessoreinheit 130 vorhanden, die über Verbindungsleitungen 115, 116, 117, 119 mit der Bewegungsvorrichtung 110, der flächigen und/oder punktförmigen Beleuchtungsvorrichtung 113, 118 und der Kamera 140 verbunden ist. Dazu können elektrische und/oder optische Verbindungsleitungen 115, 116, 117, 119 verwendet werden. Selbstverständlich können zur Übertragung von Steuersignalen und Bilddaten die einzelnen Teile der Vorrichtung 100 auch über kabellose Verbindungen miteinander kommunizieren, wobei jeder Teil der Vorrichtung 100 eine eigene Energieversorgung benötigt. Die von der Kamera 140 erzeugten digitalen Aufnahmen werden an die Prozessoreinheit 130 übermittelt und durch diese verarbeitet und ausgewertet. Je nach Ergebnis der Auswertung kann die Prozessoreinheit 130 den Antrieb der Bewegungsvorrichtung 110 erneut aktivieren, um die noch vorhandene feste Substanz 153 aufzulösen oder erneut kinetische Energie dem Gemisch beziehungsweise der Lösung zuführen, um eine weitere Serie von digitalen Aufnahmen zu Kontrollzwecken aufzunehmen. Ferner kann auf der Basis mehrerer ausgewerteter Serien abgeschätzt werden, wie lange der aktuelle Auflösungsprozess noch dauern könnte.

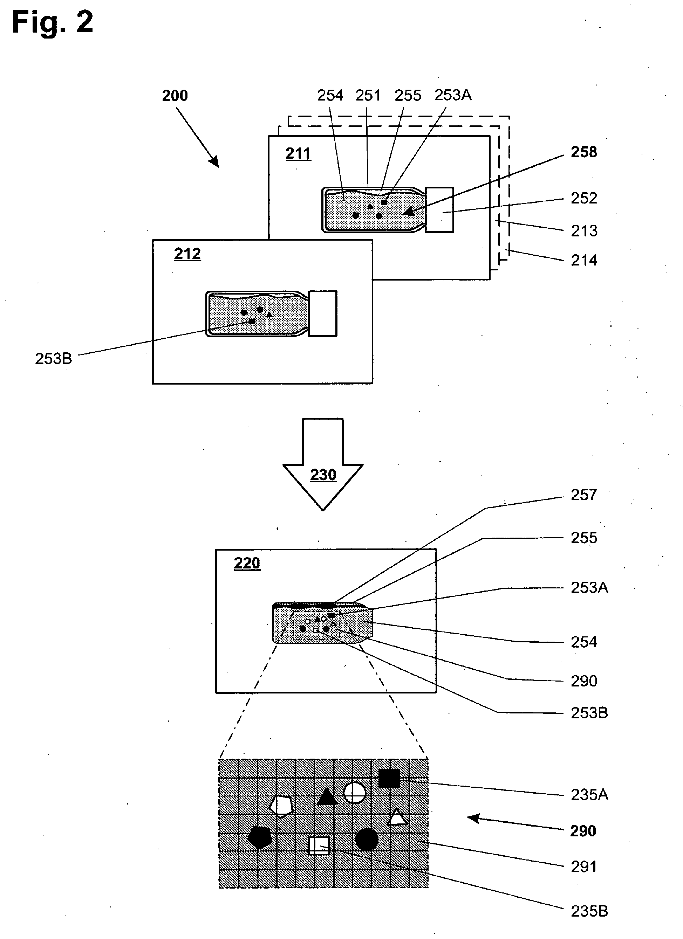

Die Verarbeitung der von der Kamera 140 erzeugten digitalen Aufnahmen ist schematisch in Figur 2 dargestellt. Mit Hilfe der Kamera 140 wird in der weiter oben beschriebenen Aufnahmephase mindestens eine Serie 200 von mindestens zwei digitalen Aufnahmen 211, 212 der transparenten Stelle 251, beziehungsweise des an dieser Stelle einsehbaren bewegten Gemischs 258 gemacht. Auf der ersten digitalen Aufnahme 211 ist das Gemisch 258 aus fester Substanz 253A und flüssiger Phase 254, das Gasvolumen 255, die als flaschenförmiger Körper ausgebildete transparente Stelle 251 und der Behälterverschluss 252 erkennbar. Exakt dieselben Elemente sind auch auf der zweiten digitalen Aufnahme 212 abgebildet. Der einzige Unterschied zwischen den beiden digitalen Aufnahmen 211, 212 besteht darin, dass sich auf der zweiten digitalen Aufnahme 212 die Positionen der Partikel der festen Substanz 253B zu den Positionen der Partikel der festen Substanz 253A der ersten digitalen Aufnahme 211 verändert haben. Die einzelnen Partikel der festen Substanz 253A, 253B sind auf den beiden Aufnahmen 211, 212 als Kontrastflächen erkennbar und weisen zur als Grundfläche erkennbaren flüssigen Phase 254 unterschiedliche Pixelwerte auf.

In der schematisch als Pfeil dargestellten Prozessoreinheit 230 werden die Pixelwerte der zweiten Aufnahme 212 von den Pixelwerten der ersten Aufnahme 211 subtrahiert und mit den aus der Subtraktion resultierenden Pixelwerten wird ein Analysebild 220 erzeugt.

Das Analysebild 220 weist, sofern keine Überdeckung der Positionen einzelner Kontrastflächen vorhanden ist, aufgrund der Differenzbildung die doppelte Anzahl an Kontrastflächen auf. Die Kontrastflächen der festen Substanz 253A der ersten Aufnahme 211 heben sich dunkel von der Grundfläche der flüssigen Phase 254 ab. Auch die sich überschneidende Kontur des Flüssigkeitsspiegels 257 zwischen dem Gasvolumen 255 und der flüssigen Phase 254 hebt sich dunkel ab. Die Kontrastflächen der festen Substanz 253B der zweiten Aufnahme 212 heben sich hell von der Grundfläche der flüssigen Phase 254 ab. Damit die überschneidende Kontur des Flüssigkeitsspiegels 257 nicht in die Auswertung einfliesst, kann aus dem Analysebild 220 ein Auswertungsbereich 290 ausgewählt werden, wobei nur die innerhalb dieses Auswertungsbereichs 290 vorhandenen Kontrastflächen ausgewertet werden. Selbstverständlich kann die Auswahl des Auswertungsbereichs 290 auch vor der Berechnung des Analysebildes 220 erfolgen, wobei die entsprechenden Ausschnitte aus den digitalisierten Aufnahmen 211, 212 ausgewählt werden und nur die Pixelwerte dieser Ausschnitte verrechnet werden. Dieses Vorgehen ist insbesondere beim Einsatz von Prozessoreinheiten 130 mit geringer Rechenleistung und geringen Speicherkapazitäten von Vorteil.

Anstelle der vorangehend beschriebenen Differenzbildung, kann aus der ersten digitalisierten Aufnahme 211 und weiteren digitalisierten Aufnahmen 213, 214 derselben Serie 200 ein Durchschnittsbild berechnet werden. Anschliessend wird das Analysebild 220 durch die Subtraktion der Pixelwerte des Durchschnittsbildes von den Pixelwerten der zweiten digitalisierten Aufnahme 212 erzeugt. Auch auf diesem Analysebild 220 sind die sich bewegenden Partikel 253A, 253B des Gemischs als Kontrastflächen beziehungsweise als unterschiedliche Pixelwerte erkennbar.

Eine weitere mögliche Methode besteht darin, das Analysebild 220 mittels einer Bewegungsschätzung der einzelnen Pixel mindesten zweier digitalisierter Aufnahmen 211, 212 zu erzeugen. Durch die Bewegungsschätzung werden alle Pixel als statischer Bildanteil definiert, wobei deren Bewegungsvektoren in den Aufnahmen 211, 212 zueinander null sind. Das heisst, dass mit erheblich grösserem Rechenaufwand die Positionen der einzelnen Pixel und deren Pixelwerte mit den umliegenden Pixel und deren Pixelwerten verglichen werden muss. Auch auf diesem Analysebild 220 sind die als dynamischer Bildanteil sich bewegenden Partikel der festen Substanz 253A, 253B des Gemischs als Kontrastflächen beziehungsweise als unterschiedliche Pixelwerte erkennbar.

Anschliessend wird der Auswertungsbereich 290 in Segmentflächen 291 gleicher Grösse beziehungsweise Fläche segmentiert. Selbstverständlich können die Segmentflächen 291 auch unterschiedliche Grösse aufweisen und einzelne Segmentflächen in der weiteren Auswertung unberücksichtigt bleiben. Dies kann notwendig sein, wenn beispielsweise einzelne Stellen des Behälters die elektromagnetischen Wellen reflektieren, diese Stellen im Auswertungsbereich 290 liegen und somit "blinde Stellen" sind.

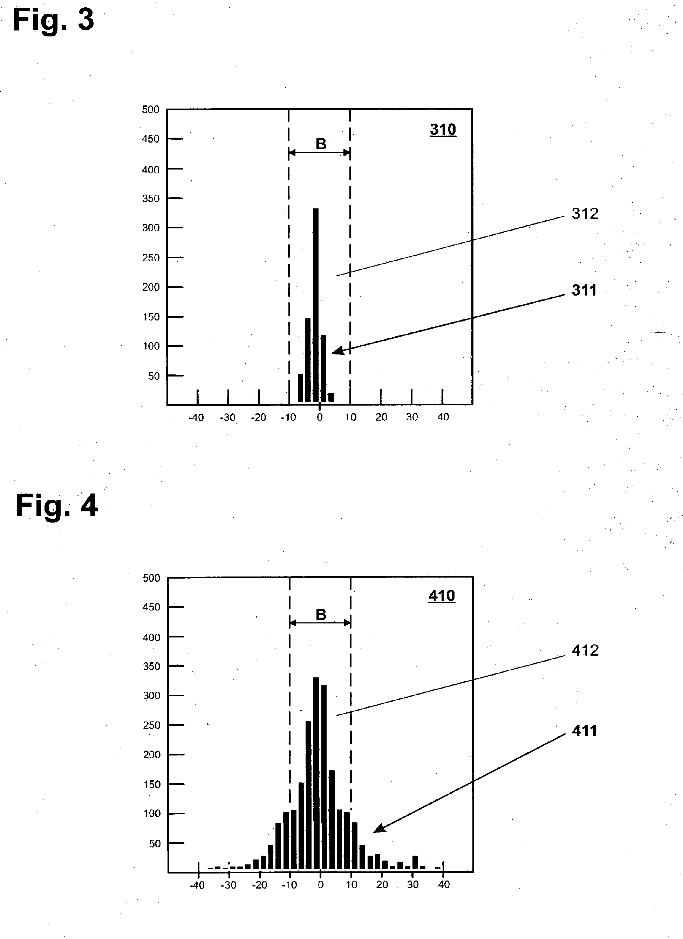

Die weitere Auswertung der einzelnen Segmentflächen 291 zeigen die Figuren 3 und 4. Die Figur 3 zeigt in der Darstellung eines ersten Histogramms 310 eine erste Häufigkeitsverteilung 311 von Pixelwerten einer Segmentfläche, wobei aufgrund der vorhandenen Pixelwerte diese Segmentfläche keine Kontrastflächen aufweist. In der Abszisse sind die vom mittleren Pixelwert null (grau) abweichenden positiven (hellen) und negativen (dunklen) Pixelwerte aufgetragen, in der Ordinate die Anzahl der Pixelwerte mit derselben Pixelwertgrösse. Jede Kamera beziehungsweise deren Sensorelement erzeugt als digitale Aufnahme eine Reihe von Pixelwerten. Selbst bei der Aufnahme einer gleichmässig ausgeleuchteten, monochromen Fläche weichen in der Regel einige Pixelwerte von den eigentlich erwarteten, gleichen Pixelwerten ab. Diese Abweichungen werden als kamerabedingtes Rauschen bezeichnet und hängen im Wesentlichen von der Qualität der Kamera, aber auch von der Ausleuchtung des aufzunehmenden Objektes ab. Jeder Schatten einer Flächen- Unebenheit oder infolge mangelhafter Beleuchtung tritt auf der digitalisierten Aufnahme logischerweise als schwach sichtbare Kontrastfläche in Erscheinung.

Um das kamerabedingte Rauschen in der Auswertung zu eliminieren, wird wie in Figur 3 dargestellt, eine Bandbreite 312 von Pixelwerten der ersten Häufigkeitsverteilung 311 von der weiteren Auswertung ausgeschlossen. Die vorbestimmte Bandbreite 312 mit der Breite B ist somit auch ein Mass für die Empfindlichkeit und Funktionssicherheit der Vorrichtung. Selbstverständlich kann auch eine schmälere Bandbreite 312 definiert werden und dafür eine gewisse maximale Anzahl von Pixelwerten ausserhalb der Bandbreite 312 zugelassen werden. Es empfiehlt sich dann, das Mass der Anzahl Pixelwerte ausserhalb der Bandbreite 312 relativ zur gesamten Zahl der Pixel der Segmentfläche 291 zu bestimmen.

Die Figur 4 zeigt in der Darstellung eines Histogramms 410 eine zweite Häufigkeitsverteilung 411 von Pixelwerten einer Segmentfläche. Anders als in der Figur 3, ist die zweite Häufigkeitsverteilung 411 in der Figur 4 wesentlich breiter, wodurch auch Pixelwerte ausserhalb der vorbestimmten Bandbreite 412 vorhanden sind. Dies deutet darauf hin, dass die betrachtete Segmentfläche Kontrastflächen aufweist. Nun können die Anzahl Segmentflächen mit Kontrastflächen gezählt werden, wobei diese Anzahl Segmentflächen ein Mass für die vorhandene Menge fester Substanz und/oder für den Fortschritt des Auflösungsprozesses darstellt.

Im Falle der Überwachung eines Auflösungsprozesses kann anhand dieser Erkenntnisse und unter Berücksichtigung der Stoffeigenschaften des Gemischs abgeschätzt werden, wie lange das Gemisch noch geschüttelt werden muss, bis die feste Substanz vollständig in Lösung übergegangen ist.

Durch den Vergleich mehrerer Analysebilder in ihrer chronologischen Reihenfolge kann die Verminderung der festen Substanz und die Verkleinerung der Partikelgrösse im Gemisch des Behälters beobachtet und ausgewertet werden. Durch Extrapolation dieser Ergebnisse, gegebenenfalls unter Zuhilfenahme von Erfahrungswerten, kann beispielsweise die noch anstehende Auflösungszeit ermittelt werden.

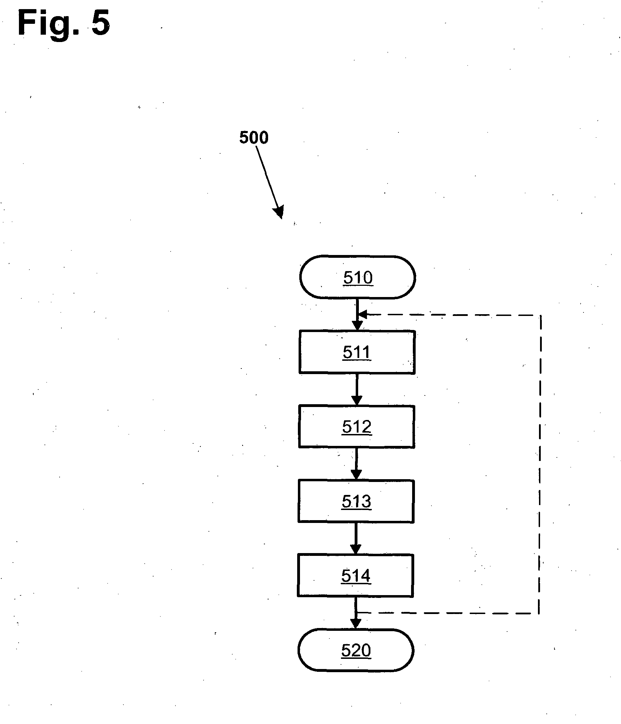

Ein Flussdiagramm 500 mit den wesentlichen Verfahrensschritten des erfindungsgemässen Verfahrens ist in Figur 5 dargestellt. Dabei findet beispielsweise eine betriebsbereite Vorrichtung 100 Verwendung, wie sie in der Figur 1 dargestellt ist. Nachfolgend sind in der Beschreibung die Vorrichtungsmerkmale mit den Bezugszeichen der Figur 1, die Verarbeitung der digitalisierten Aufnahmen mit den Bezugszeichen der Figur 2 und die Verfahrensschritte mit den Bezugszeichen der Figur 5 versehen. Ausgehend vom Start 510 wird in einem ersten Schritt 511 der Behälter 150 mittels der Bewegungsvorrichtung 110 bewegt und dem Gemisch 258 dadurch kinetische Energie zugeführt. Anschliessend wird im zweiten Schritt 512 der Behälter 150 durch das Abschalten des Antriebs der Bewegungsvorrichtung 110 in eine Aufnahmephase gebracht und mindestens eine Serie 200 mit mindestens zwei digitalisierten Aufnahmen 211, 212 des im ruhenden Behälter 150 bewegten Gemischs 258 gemacht. Im dritten Schritt 513 wird durch die Subtraktion zweier digitalisierter Aufnahmen 211, 212 derselben Serie ein Analysebild 220 berechnet, wobei die sich bewegenden Teile der festen Substanz 153, 253A, 253B als Kontrastflächen beziehungsweise unterschiedliche Pixelwerte erkennbar sind. Alternativ zur Subtraktion zweier digitalisierter Aufnahmen 211, 212 kann zur Erzeugung des Analysebildes 220 auch die weiter oben beschriebenen Methoden mit einen Durchschnittsbild oder mit der Bewegungsschätzung angewendet werden.

Im vierten Schritt 514 wird das Analysebild 220 auf das Vorhandensein von Kontrastflächen beziehungsweise unterschiedliche Pixelwerte untersucht. Mit dem Abschluss der Untersuchung des Analysebildes 220 ist das Ende 520 der Abfolge der absolut notwendigen Verfahrensschritte erreicht. Sofern bei der Untersuchung des Analysebildes 220 Kontrastflächen gefunden werden und es sich um einen Auflösungsprozess handelt, können die vorangehenden Verfahrensschritte 511, 512, 513, 514 solange wiederholt werden, bis keine Kontrastflächen mehr detektiert werden können. Diese Wiederholschleife ist mittels der unterbrochenen Linie dargestellt.

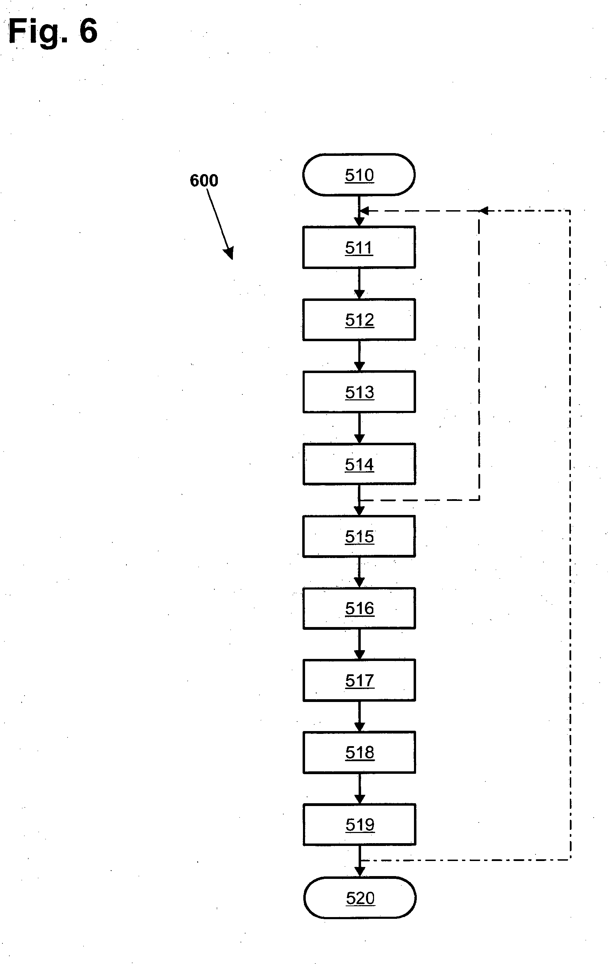

Wie in der Figur 6 dargestellt ist, kann die Untersuchung des Analysebildes 220 in weitere Verfahrenschritte aufgeteilt sein. Die Figur 6 zeigt ein ausführliches Flussdiagramm 600 des erfindungsgemässen Verfahrens mit weiteren möglichen Verfahrensschritten. Das ganze Verfahren kann in einem Computerprogramm oder in einer Firmware abgebildet sein, so dass das programmierte Verfahren Schritt für Schritt durch die Prozessoreinheit abgearbeitet werden kann. Die in Zusammenhang mit der Figur 5 bereits erörterten Schritte und Vorrichtungsmerkmale weisen dieselben Bezugszeichen auf und werden nicht mehr erklärt.

Eine erste Ergänzung bildet ein fünfter Schritt 515, bei dem aus dem Analysebild 220 mindestens ein Auswertungsbereich 290 ausgewählt wird damit störende Randbereiche wie das Gasvolumen 255 oder der Flüssigkeitsspiegel 257 die Auswertung nicht negativ beeinflussen. Dieser Auswertungsbereich 290 wird in einem sechsten Schritt 516 in Segmentflächen 291 gleicher Grösse segmentiert. Hernach wird in einem siebten Schritt 517 jede Segmentfläche 291 auf das Vorhandensein von Kontrastflächen beziehungsweise auf unterschiedliche Pixelwerte untersucht. Dies geschieht dadurch, dass innerhalb einer Segmentfläche die Häufigkeitsverteilung 311, 411 der Pixelwerte ausgewertet wird und zur Eliminierung kamerabedingten Rauschens eine vorbestimmte Pixelwert- Bandbreite 312, 412 der Häufigkeitsverteilung 311, 411 in einer weiteren Auswertung nicht berücksichtigt wird.

In einem achten Schritt 518 kann nun die Anzahl Segmentflächen 291 festgestellt werden, deren Häufigkeitsverteilung 411 von der vorbestimmten Pixelwert- Bandbreite 412 abweichende Pixelwerte aufweisen. Die Anzahl der Segmentflächen 291 stellt bereits ein Mass für die vorhandene Menge fester Substanz und/oder für den Fortschritt des Auflösungsprozesses dar.

Eine noch präzisere Auswertung lässt sich in einem neunten Schritt 519 erlangen, wenn die Segmentflächen 291, deren Häufigkeitsverteilung 411 von der vorbestimmten Pixelwert- Bandbreite 412 abweichende Pixelwerte aufweisen, nach einem vorgegebenen Modus gewichtet werden. Die Gewichtung kann beispielsweise dadurch erfolgen, dass die jeweilige Anzahl eines entsprechenden Pixelwertes mit einem Faktor multipliziert wird und dieser Faktor von der Entfernung des entsprechenden Pixelwerts zum Pixelwert Null abhängt. Durch die Gewichtung werden beispielsweise sehr dunkle und sehr helle Kontrastflächen stärker berücksichtigt, weil diese auf grosse Partikel hinweisen. Durch die Grösse der Partikel werden der Auflösungsprozess und insbesondere die dazu benötigte Zeit bekanntlich stark beeinflusst. Die gewichteten Werte der Segmentflächen 291 können anschliessend addiert werden, wobei die Summe der Werte wiederum ein Mass für die vorhandene Menge fester Substanz und/oder für den Fortschritt des Auflösungsprozesses darstellt. Selbstverständlich kann basierend auf dieser präziseren Auswertung das weitere Verfahren festgelegt werden. Beispielsweise kann abgeschätzt werden, wie lange noch geschüttelt werden muss, bis keine Kontrastflächen mehr detektiert werden können. Infolgedessen werden die vorangehenden Verfahrensschritte 511, 512, 513, 514, 515, 516, 517 518, 519 nochmals wiederholt um eine Endkontrolle der Lösung durchzuführen. Diese Wiederholschleife ist mittels der strichpunktierten Linie dargestellt.

Obwohl die Erfindung durch die Darstellung spezifischer Ausführungsbeispiele beschrieben worden ist, ist es offensichtlich, dass diese in Kenntnis ihrer technischen Lehre auch in weiteren Einsatzgebieten verwendet werden kann. Beispielsweise kann die erfindungsgemässe Vorrichtung und das erfindungsgemässe Verfahren auch zur Überwachung des Mischvorganges zweier oder mehrerer Flüssigkeiten verwendet werden, wenn die zu mischenden Flüssigkeiten voneinander unterscheidbare Farben aufweisen und/oder deren Brechungsindex verschieden ist, so dass die als Schlieren erkennbaren ungemischten Bereiche durch die Kamera erfassbar sind. Der Ersatz der in der vorliegenden Schrift erwähnten festen Substanz durch eine weitere flüssige Phase mit optisch unterscheidbaren Eigenschaften zur flüssigen Phase ist deshalb innerhalb des Schutzbereichs der unabhängigen Ansprüche.

### Bezugszeichenliste

- 100: Vorrichtung
- 110: Bewegungsvorrichtung
- 111: Aufnahme
- 112: Aufnahmewelle
- 113: flächige Beleuchtungsvorrichtung
- 119,117,116,115: Verbindungsleitung
- 118: punktförmige Beleuchtungsvorrichtung/Spot
- 230, 130: Prozessoreinheit
- 140: Kamera
- 141: Objektiv
- 150: Behälter
- 151: transparente Stelle
- 152: Behälterverschluss
- 153: feste Substanz, Partikel der festen Substanz
- 154: flüssige Phase
- 155: Gasvolumen
- 171: oszillierende Bewegung in Längsrichtung
- 172: Drehbewegung
- 175,174,173: Lichtwellen
- 200: Serie von Aufnahmen
- 211: erste digitale Aufnahme
- 212: zweite digitale Aufnahme
- 214, 213: weitere digitale Aufnahme
- 220: Analysebild
- 251: Aufnahme der transparenten Stelle
- 252: Aufnahme des Behälterverschlusses
- 253B, 253A: Aufnahme der festen Substanz / Partikel der festen Substanz
- 254: Aufnahme der flüssigen Phase
- 255: Aufnahme des Gasvolumens
- 257: Aufnahme des Flüssigkeitsspiegels
- 258: Aufnahme des Gemischs
- 290: Auswertungsbereich
- 291: Segmentfläche
- 310: erstes Histogramm
- 311: erste Häufigkeitsverteilung
- 412, 312: vorbestimmte Bandbreite
- 410: zweites Histogramm
- 411: zweite Häufigkeitsverteilung
- 600, 500: Flussdiagramm des Verfahrens
- 510: Start
- 511: erster Schritt: kinetische Energie zuführen
- 512: zweiter Schritt: Aufnahmephase und Serie von digit. Aufnahmen
- 513: dritter Schritt: Analysebild berechnen
- 514: vierter Schritt: Analysebild untersuchen
- 515: fünfter Schritt: Auswertungsbereich auswählen
- 516: sechster Schritt: Segmentieren des Auswertungsbereichs
- 517: siebter Schritt: Segmentfläche untersuchen
- 518: achter Schritt: Anzahl Segmentflächen mit Kontrastflächen
- 519: neunter Schritt: Gewichtung der Segmentflächen
- 520: Ende

## Patentansprüche

1. Vorrichtung (100) zur Erkennung fester Substanzen (153) in einer flüssigen Phase (154), wobei die feste Substanz (153) und die flüssige Phase (154) als Gemisch in einem zumindest teilweise transparenten Behälter (150) eingeschlossen sind und die Vorrichtung (100) eine Prozessoreinheit (130, 230) und eine Kamera (140) zur Erzeugung digitalisierter Aufnahmen (211, 212, 213, 214) als Arrays von Pixeln mit ihren zugehörenden Pixelwerten umfasst, wobei die Vorrichtung (100) ferner eine Bewegungsvorrichtung (110) zur Aufnahme des Behälters (150) aufweist, mittels welcher Bewegungsvorrichtung (110) der Behälter (150) bewegbar und dem Gemisch dadurch kinetische Energie zuführbar ist, wobei die Kamera (140) zur bildlichen Erfassung des bewegten Gemischs auf eine transparente Stelle (151) des Behälters (150) gerichtet ist und die mittels der Kamera (140) während einer Aufnahmephase erfassten, digitalisierten Aufnahmen (211, 212, 213, 214) des bewegten Gemischs an die Prozessoreinheit (130, 230) übermittelbar sind, **dadurch gekennzeichnet, dass** der Behälter (150) im Betriebszustand der Vorrichtung (100) mit einer Aufnahme (111) der Bewegungsvorrichtung (110) lösbar verbunden ist und die Aufnahme (111) der Bewegungsvorrichtung (110) über eine Aufnahmewelle (112) mit einem Antrieb verbunden ist und mittels des Antriebs an der Aufnahmewelle (112) oszillierende Bewegungen (171) in deren Längsrichtung erzeugbar sind.

2. Vorrichtung (100) nach Anspruch 1, **dadurch gekennzeichnet, dass** mindestens eine Serie (200) umfassend mindestens zwei digitalisierte Aufnahmen (211, 212, 213, 214) des bewegten Gemisches vorhanden ist und aus mindestens zwei digitalisierten Aufnahmen (211, 212, 213, 214) ein Analysebild (220) des bewegten Gemisches erzeugbar ist.

3. Vorrichtung (100) nach Anspruch 2, **dadurch gekennzeichnet, dass** das Analysebild (220) durch die Subtraktion der Pixelwerte einer ersten digitalisierten Aufnahme (211) von den Pixelwerten einer zweiten digitalisierten Aufnahme (212) erzeugt ist, auf welchem Analysebild (220) die sich bewegenden festen Substanzteile (153) des Gemischs als Kontrastflächen beziehungsweise als unterschiedliche Pixelwerte erkennbar sind.

4. Vorrichtung (100) nach Anspruch 2, **dadurch gekennzeichnet, dass** ein Durchschnittsbild aus einer Serie vorangehender digitalisierter Aufnahmen (211, 212, 213, 214) berechnet ist, dass das Analysebild (220) durch die Subtraktion der Pixelwerte des Durchschnittsbildes von den Pixelwerten einer digitalisierten Aufnahme (211, 212, 213, 214) erzeugt ist, und dass auf dem Analysebild (220) die sich bewegende feste Substanz (153) des Gemischs als Kontrastflächen beziehungsweise als unterschiedliche Pixelwerte erkennbar sind.

5. Vorrichtung (100) nach Anspruch 2, **dadurch gekennzeichnet, dass** das Analysebild (220) mittels einer Bewegungsschätzung der einzelnen Pixel, mindesten zweier digitalisierter Aufnahmen (211, 212, 213, 214) erzeugt ist, wobei durch die Bewegungsschätzung alle Pixel als statischer Bildanteil definiert sind, deren Bewegungsvektoren in den Aufnahmen (211, 212, 213, 214) zueinander Null sind, wobei auf dem Analysebild (220) die als dynamischer Bildanteil sich bewegenden festen Substanzteile (153) des Gemischs als Kontrastflächen beziehungsweise als unterschiedliche Pixelwerte erkennbar sind.

6. Vorrichtung (100) nach einem der Ansprüche 3 bis 5, **dadurch gekennzeichnet, dass** aus dem Analysebild (220) mindestens ein Auswertungsbereich (290) ausgewählt ist.

7. Vorrichtung (100) nach Anspruch 6, **dadurch gekennzeichnet, dass** der mindestens eine Auswertungsbereich (290) in Segmentflächen (291) gleicher oder unterschiedlicher Grösse segmentiert ist.

8. Vorrichtung (100) nach einem der Ansprüche 1 bis 7, **dadurch gekennzeichnet, dass** die Häufigkeitsverteilung (311, 411) der Pixelwerte innerhalb eines Analysebildes (220) oder einer Segmentfläche (291) ausgewertet sind und zur Eliminierung kamerabedingten Rauschens eine vorbestimmte Bandbreite (312, 412) von Pixelwerten der Häufigkeitsverteilung (311 , 411) in einer weiteren Auswertung nicht berücksichtigt ist.

9. Vorrichtung (100) nach einem der Ansprüche 1 bis 8, **dadurch gekennzeichnet, dass** diese mindestens eine flächige und/oder punktförmige Beleuchtungsvorrichtung (113, 118) aufweist, wobei die elektromagnetischen Wellen beziehungsweise die Lichtwellen (173) der mindestens einen Beleuchtungsvorrichtung (113, 118) auf die von der Kamera (140) zu erfassende, transparente Stelle (151) des Behälters (150) gerichtet ist.

10. Verfahren (500, 600) zur Erkennung fester Substanzen (153) in einer flüssigen Phase (154), wobei die feste Substanz (153) und die flüssige Phase (154) als Gemisch in einem zumindest teilweise transparenten Behälter (150) eingeschlossen sind, umfassend die Schritte (511, 512, 513, 514, 515, 516, 517, 518, 519), dass
• der Behälter (150) bewegt und dem Gemisch dadurch kinetische Energie zugeführt wird,
• mittels einer Kamera (140) in einer Aufnahmephase mindestens eine Serie (200) mit mindestens zwei digitalisierten Aufnahmen (211, 212, 213, 214) des im Behälter (150) bewegten Gemischs als Arrays von Pixeln mit ihren zugehörenden Pixelwerten gemacht werden,
• aus den Arrays von Pixeln mit ihren zugehörenden Pixelwerten ein Analysebild (220) berechnet wird, wobei die sich bewegende feste Substanz (153) als Kontrastflächen beziehungsweise unterschiedliche Pixelwerte erkennbar sind und
• das Analysebild (220) auf das Vorhandensein von Kontrastflächen beziehungsweise unterschiedliche Pixelwerte untersucht wird, **dadurch gekennzeichnet, dass** der Behälter (150) im Betriebszustand der Vorrichtung (100) mit einer Aufnahme (111) der Bewegungsvorrichtung (110) lösbar verbunden ist und die Aufnahme (111) der Bewegungsvorrichtung (110) über eine Aufnahmewelle (112) mit einem Antrieb verbunden ist und mittels des Antriebs an der Aufnahmewelle (112) oszillierende Bewegungen (171) in deren Längsrichtung erzeugbar sind.

11. Verfahren (500, 600) nach Anspruch 10, **gekennzeichnet durch** den weiteren Schritt (515), dass aus dem Analysebild (220) mindestens ein Auswertungsbereich (290) ausgewählt wird.

12. Verfahren (500, 600) nach Anspruch 10 oder 11, **gekennzeichnet durch** die weiteren Schritte (516), dass das Analysebild (220) oder der mindestens eine Auswertungsbereich (290) in Segmentflächen (291) gleicher Grösse segmentiert wird und jede Segmentfläche (291) auf das Vorhandensein von Kontrastflächen beziehungsweise auf unterschiedliche Pixelwerte untersucht wird.

13. Verfahren (500, 600) nach Anspruch 12, **dadurch gekennzeichnet, dass** innerhalb eines Analysebildes (220) oder einer Segmentfläche (291) die Häufigkeitsverteilung (311, 411) der Pixelwerte ausgewertet wird und zur Eliminierung kamerabedingten Rauschens eine vorbestimmte Pixelwert-Bandbreite (312, 412) der Häufigkeitsverteilung (311, 411) in einer weiteren Auswertung nicht berücksichtigt wird.

14. Verfahren (500, 600) nach Anspruch 13, **gekennzeichnet durch** die weiteren Schritte (517, 518), dass die Anzahl Segmentflächen (291) festgestellt wird, deren Häufigkeitsverteilung (311, 411) von der vorbestimmten Pixelwert-Bandbreite (312, 412) abweichende Pixelwerte aufweisen, wobei diese Anzahl der Segmentflächen (291) ein Mass für die vorhandene Menge fester Substanz (153) und/oder für den Fortschritt des Auflösungsprozesses darstellt.

15. Verfahren (500, 600) nach Anspruch 13, **gekennzeichnet durch** die weiteren Schritte (517, 519), dass die Segmentflächen (291), deren Häufigkeitsverteilung (311, 411) von der vorbestimmten Pixelwert- Bandbreite (312, 412) abweichende Pixelwerte aufweisen, nach einem vorgegebenen Modus gewichtet und die gewichteten Werte der Segmentflächen (291) addiert werden, wobei die Summe der Werte ein Mass für die vorhandene Menge fester Substanz (153) und/oder für den Fortschritt des Auflösungsprozesses darstellt.

## Claims

1. Apparatus (100) for the detection of solid substances (153) in a liquid phase (154), wherein the solid substance (153) and the liquid phase (154) are enclosed as a mixture in an at least partially transparent container (150) and the apparatus (100) comprises a processor unit (130, 230) and a camera (140) to make digitized images (211, 212, 213, 214) in the form of arrays of pixels with their associated pixel values, **characterized in that** the apparatus (100) further comprises an agitator device (110) designed to hold the container (150) and operable to impart movements to the container (150) and to thereby inject kinetic energy into the mixture and in the operating state of the apparatus (100), the container (150) is oriented with its length extending in the horizontal direction and is releasably connected to a holder socket (111) of the agitator device (110) and the holder socket (111) is connected by way of a holder shaft (112) to a drive mechanism, wherein the drive mechanism can generate oscillatory movements (171) in the holder shaft 112 in its lengthwise direction and further **characterized in that** the camera (140) is aimed at a transparent portion (151) of the container (150) to take images of the mixture in motion, and that the digitized images (211, 212, 213, 214) of the mixture in motion that are taken by the camera (140) during a recording phase are capable of being transmitted to the processor unit (130, 230).

2. Apparatus (100) according to claim 1, **characterized by** the presence of at least one series (200) of at least two digitized images (211, 212, 213, 214) of the mixture in motion, and further **characterized by** the capability to generate an analytical representation (220) of the mixture in motion on the basis of at least two digitized images (211, 212, 213, 214).

3. Apparatus (100) according to claim 2, **characterized in that** the analytical representation (220) can be generated by subtracting the pixel values of a first digitized image (211) from the pixel values of a second digitized image (212), so that in said analytical representation (220) the moving solid substance particles (153) of the mixture will appear as contrast areas, i.e. as areas distinguished by having different pixel values.

4. Apparatus (100) according to claim 2, **characterized in that** an intersection of a series of preceding digitized images (211, 212, 213, 214) is calculated, that the analytical representation (220) is generated by subtracting the pixel values of the intersection from the pixel values of a digitized image (211, 212, 213, 214), and that the moving solid substance (153) of the mixture appears in the analytical representation (220) as contrast areas, i.e. as areas distinguished by having different pixel values.

5. Apparatus (100) according to claim 2, **characterized in that** the analytical representation (220) can be generated by means of an estimate of the movements of the individual pixels of at least two digitized images (211, 212, 213, 214), wherein the estimate of the movements will characterize as the static image portion all those pixels whose movement vectors from one to another of the images (211, 212, 213, 214) are zero, and wherein the moving solid substance parts (153) of the mixture that make up the dynamic image portion appear in the analytical representation (220) as contrast areas, i.e. as areas distinguished by having different pixel values.

6. Apparatus (100) according to one of the claims 3 to 5, **characterized in that** at least one evaluation zone (290) is selected from the analytical representation (220).

7. Apparatus (100) according to claim 6, **characterized in that** the at least one evaluation zone (290) is subdivided into segments (291) of equal or different size.

8. Apparatus (100) according to one of the claims 1 to 7, **characterized in that** the frequency distribution (311, 411) of the pixel values within an analytical representation (220) or a segment area (291) are evaluated and that, in order to eliminate camera-related noise, a predetermined bandwidth (312, 412) of pixel values of the frequency distribution (311, 411) is dropped from consideration in a continued evaluation.

9. Apparatus (100) according to one of the claims 1 to 8, **characterized in that** the apparatus (100) comprises at least one area light source (113) and/or point light source (118), wherein the electromagnetic waves, specifically the light waves (173) of the at least one light source (113, 118) are aimed at the transparent portion (151) of the container (150) that is to be captured by the camera (140).

10. Method (500, 600) for the detection of solid substances (153) in a liquid phase (154), wherein the solid substance (153) and the liquid phase (154) are enclosed as a mixture in an at least partially transparent container (150), **characterized by** the steps (511, 512, 513, 514, 515, 516, 517, 518, 519) according to which:
- the container (150) is set in motion, whereby kinetic energy is injected into the mixture;
- in a recording phase, using a camera (140), at least one series (200) of at least two digitized images (211, 212) in the form of pixel arrays with associated pixel values is taken of the mixture (258) moving around inside the container (150);
- from the arrays of pixels with their associated pixel values, an analytical representation (220) is calculated, wherein the moving solid substance (153) appears as contrast areas, i.e. as areas distinguished by having different pixel values; and
- the analytical representation (220) is examined for the presence of contrast areas, i.e. areas distinguished by having different pixel values.

11. Method (500, 600) according to claim 10, **characterized by** the further step (515) that at least one evaluation zone (290) is selected from the analytical representation (220).

12. Method (500, 600) according to claim 10 or 11, **characterized by** the further step (516) that the analytical representation (220) or the at least one evaluation zone (290) is subdivided into segments (291) of equal size, and that each segment (291) is examined for the presence of contrast areas, i.e. areas distinguished by having different pixel values.

13. Method (500, 600) according to claim 12, **characterized in that** within an analytical representation (220) or a segment (291) the frequency distribution (311, 411) of the pixel values is evaluated and that, in order to eliminate camera-related noise, a predetermined bandwidth (312, 412) of pixel values of the frequency distribution (311, 411) is dropped from consideration in a continuing evaluation.

14. Method (500, 600) according to claim 13, **characterized by** the further steps (517, 518) in which the number of segment areas (291) is registered whose frequency distributions (311, 411) comprise pixel values that fall outside the predetermined bandwidth (312, 412), wherein said number of segment areas (291) represents a measure for the amount of solid substance (153) that is present, and/or for the progress of the process of dissolution.

15. Method (500, 600) according to claim 13, **characterized by** the further steps (517, 519) wherein the segments (291) whose frequency distributions (311, 411) comprise pixel values falling outside the predetermined bandwidth (312, 412) are weighted according to a predetermined plan, wherein the weighted numbers of the segments (291) are added to each other, and wherein the sum of the numbers represents a measure for the amount of solid substance (153) that is present, and/or for the progress of the process of dissolution.

## Revendications

1. Dispositif (100) pour la détection de substances solides (153) dans une phase liquide (154), la substance solide (153) et la phase liquide (154) étant contenues sous forme de mélange dans un récipient (150) au moins partiellement transparent, et le dispositif (100) comprenant une unité de processeur (130, 230) et une caméra (140) permettant d'effectuer des enregistrements numériques (211, 212, 213, 214) sous la forme de réseaux de pixels avec leurs valeurs de pixel correspondantes, le dispositif (100) comportant en outre un dispositif de mise en mouvement (110) pour l'enregistrement du récipient (150), le dispositif de mise en mouvement (110) permettant de mettre en mouvement le récipient (150) et donc de transmettre une énergie cinétique au mélange, la caméra (140) étant orientée sur une zone transparente (151) du récipient (150) pour l'imagerie du mélange mis en mouvement, et les enregistrements numérisés (211, 212, 213, 214) du mélange mis en mouvement, enregistrés à l'aide de la caméra (140) pendant une phase d'enregistrement, pouvant être transmis à l'unité de processeur (130, 230), **caractérisé en ce que** dans l'état de fonctionnement du dispositif (100), le récipient (150) est relié de façon détachable à une admission (111) du dispositif de mise en mouvement (110), et l'admission (111) du dispositif de mise en mouvement (110) est reliée à un entraînement par le biais d'un arbre d'admission (112), des mouvements d'oscillation (171) pouvant être créés sur l'arbre d'admission (112) à l'aide de l'entraînement, dans le sens longitudinal de celui-ci.

2. Dispositif (100) selon la revendication 1, **caractérisé en ce qu'**il est prévu au moins une série (200) comprenant au moins deux enregistrements numériques (211, 212, 213, 214) du mélange mis en mouvement, et **en ce qu'**une image d'analyse (220) du mélange mis en mouvement peut être créée à partir d'au moins deux enregistrements numériques (211, 212, 213, 214).

3. Dispositif (100) selon la revendication 2, **caractérisé en ce que** l'image d'analyse (220) est produite par la soustraction des valeurs de pixel d'un premier enregistrement numérique (211) à partir des valeurs de pixel d'un deuxième enregistrement numérique (212), les fragments de substance solides (153) en mouvement du mélange pouvant être détectés sur l'image d'analyse (220) en tant que surfaces de contraste ou en tant que valeurs de pixel différentes.

4. Dispositif (100) selon la revendication 2, **caractérisé en ce qu'**une image moyenne est calculée à partir d'une série d'enregistrements numériques (211, 212, 213, 214) précédents, **en ce que** l'image d'analyse (220) est produite par soustraction des valeurs de pixel de l'image moyenne à partir des valeurs de pixel d'un enregistrement numérique (211, 212, 213, 214), et **en ce que** la substance solide (153) en mouvement du mélange peut être détectée sur l'image d'analyse (220) en tant que surfaces de contraste ou en tant que valeurs de pixel différentes.

5. Dispositif (100) selon la revendication 2, **caractérisé en ce que** l'image d'analyse (220) est produite au moyen d'une évaluation de mouvement des différents pixels d'au moins deux enregistrements numériques (211, 212, 213, 214), l'évaluation de mouvement permettant de définir tous les pixels en tant que parties d'image statiques, dont les vecteurs de mouvement sont nuls les uns par rapport aux autres dans les enregistrements (211, 212, 213, 214), les fragments de substance solide (153) en mouvement du mélange en tant que partie d'image dynamique pouvant être détectés sur l'image d'analyse (220) en tant que surfaces de contraste ou en tant que valeurs de pixel différentes.

6. Dispositif (100) selon l'une des revendications 3 à 5, **caractérisé en ce qu'**au moins une région d'évaluation (290) est choisie à partir de l'image d'analyse (220).

7. Dispositif (100) selon la revendication 6, **caractérisé en ce que** l'au moins une région d'évaluation (290) est segmentée en surfaces de segment (291) de taille identique ou différente.

8. Dispositif (100) selon l'une des revendications 1 à 7, **caractérisé en ce que** la distribution de fréquence (311, 411) des valeurs de pixel est évaluée dans une image d'analyse (220) ou dans une surface de segment (291), et une largeur de bande prédéfinie (312, 412) de valeurs de pixel de la distribution de fréquence (311, 411) n'est pas prise en compte dans une nouvelle évaluation pour éliminer le bruit dû à la caméra.

9. Dispositif (100) selon l'une des revendications 1 à 8, **caractérisé en ce que** celui-ci comporte au moins un dispositif d'éclairage (113, 118) surfacique et/ou ponctuel, les ondes électromagnétiques ou les ondes optiques (173) de l'au moins un dispositif d'éclairage (113, 118) étant orientées sur la zone transparente (151) du récipient (150), destinée à être enregistrée par la caméra (140).

10. Procédé (500, 600) pour la détection de substance solide (153) dans une phase liquide (154), la substance solide (153) et la phase liquide (154) étant contenues sous forme de mélange dans un récipient (150) au moins partiellement transparent, comprenant les étapes suivantes (511, 512, 513, 514, 515, 516, 517, 518, 519) :
• le récipient (150) est mis en mouvement et de l'énergie cinétique est transmise au mélange,
• au moins une série (200) avec au moins deux enregistrements numériques (211, 212, 213, 214) du mélange mis en mouvement dans le récipient (150) est constituée sous la forme d'un réseau de pixels avec ses valeurs de pixel correspondantes, à l'aide d'une caméra (140), au cours d'une phase d'enregistrement,
• une image d'analyse (220) est calculée à partir du réseau de pixels avec ses valeurs de pixel correspondantes, la substance solide (153) en mouvement pouvant être détectée en tant que surfaces de contraste ou en tant que valeurs de pixel différentes, et
• l'image d'analyse (220) est examinée quant à la présence de surfaces de contraste ou de valeurs de pixel différentes,
**caractérisé en ce que** dans l'état de fonctionnement du dispositif (100), le récipient (150) est relié de façon détachable à une admission (111) du dispositif de mise en mouvement (110), et l'admission (111) du dispositif de mise en mouvement (110) est reliée à un entraînement par le biais d'un arbre d'admission (112), des mouvements d'oscillation (171) pouvant être créés sur l'arbre d'admission (112) à l'aide de l'entraînement, dans le sens longitudinal de celui-ci.

11. Procédé (500, 600) selon la revendication 10, **caractérisé par** l'étape supplémentaire (515) consistant à choisir au moins une région d'évaluation (290) à partir de l'image d'analyse (220).

12. Procédé (500, 600) selon la revendication 10 ou 11, **caractérisé par** les étapes supplémentaires (516) consistant à segmenter l'image d'analyse (220) ou l'au moins une région d'évaluation (290) en surfaces de segment (291) de taille identique, et chaque surface de segment (291) est examinée quant à la présente de surfaces de contraste ou de valeurs de pixel différentes.

13. Procédé (500, 600) selon la revendication 12, **caractérisé en ce que** la distribution de fréquence (311, 411) des valeurs de pixel est évaluée dans une image d'analyse (220) ou dans une surface de segment (291), et une largeur de bande prédéfinie (312, 412) de valeurs de pixel de la distribution de fréquence (311, 411) n'est pas prise en compte dans une nouvelle évaluation pour éliminer le bruit dû à la caméra.

14. Procédé (500, 600) selon la revendication 13, **caractérisé par** les étapes supplémentaires (517, 518) consistant à déterminer le nombre de surfaces de segment (291), dont la distribution de fréquence (311, 411) présente des valeurs de pixel s'écartant de la largeur de bande de valeurs de pixel prédéfinie (312, 412), ce nombre de surfaces de segment (291) représentant une mesure pour la quantité de substance solide (153) présente et/ou pour la progression du processus de dissolution.

15. Procédé (500, 600) selon la revendication 13, **caractérisé par** les étapes supplémentaires (517, 519) consistant à pondérer les surfaces de segment (291) dont la distribution de fréquence (311, 411) présente des valeurs de pixel s'écartant de la largeur de bande de valeurs de pixel prédéfinie (312, 412), selon un mode prédéfini, et à additionner les valeurs pondérées des surfaces de segment (291), la somme des valeurs représentant une mesure pour la quantité de substance solide (153) présente et/ou pour la progression du processus de dissolution.
